# EUROPEAN PATENT APPLICATION

(11) **EP 4 144 356 A1**
(43) Date of publication of application: **08.03.2023**
(21) Application number: 21795270.4
(22) Date of filing: 19.04.2021
(51) Int. Cl.: A61K 35/34, A61P 3/00, A61P 3/06, A61P 3/10, A61P 5/50, A61P 21/00, A61P 31/00, C12N 5/074

(54) **STEM CELL DRUG FOR TREATING DIABETES**

(30) Priority: 26.04.2020 CN 202010340419
(71) Applicant: Soochow University, Suzhou, Jiangsu 215123 (CN)
(72) Inventor: SHI, Yufang, Suzhou, Jiangsu 215123 (CN); CAO, Lijuan, Suzhou, Jiangsu 215123 (CN); FANG, Jiankai, Suzhou, Jiangsu 215123 (CN); CHEN, Yongjing, Suzhou, Jiangsu 215123 (CN); WANG, Ying, Shanghai 200031 (CN)
(74) Representative: Santoro, Sofia
(86) International application number: PCT/CN2021/088209
(87) International publication number: WO 2021/218687

(57) **Abstract**

The use of a muscle stem cell in the preparation of a drug for preventing, alleviating and treating metabolic disorders, and a pharmaceutical composition for humans and animals, wherein the pharmaceutical composition comprises the muscle stem cell as a first active ingredient, a second active ingredient for regulating the glucose metabolism, and a pharmaceutically acceptable carrier.

## Description

### Technical field

The invention relates to an application of muscle stem cells in the preparation of drugs for the treatment of diabetes and belongs to the field of biomedical technology.

### Technical background

With the development of society and economy, the change of people's lifestyle (increase in energy intake, decrease in exercise, etc.) and the aging of the population, the incidence of type 2 diabetes mellitus is increasing year by year worldwide, especially in developing countries (it is expected to increase by 170% from now to 2025), the rate of increase will be faster, showing a strong epidemic trend. Diabetes is now the third non-communicable disease threatening the health and lives of the population, after cardiovascular diseases and neoplasms. Obesity causes the increase of adipose tissue or fatty infiltration in liver, muscle and other tissues, and the tissues of the body change from the state of inhibiting inflammation to the state of promoting inflammation, thus destroying tissue homeostasis. The occurrence of tissue inflammation will reduce the sensitivity of the tissue to insulin and produce tolerance. With increasing inflammation, insulin tolerance increases and the body loses its ability to regulate blood sugar levels, gradually developing type 2 diabetes mellitus.

Skeletal muscle is the body's largest tissue and organ, making up about 35 percent of the body's body weight. It provides the necessary power for individual movement, and also participates in the precise regulation of body energy metabolism and body temperature. When glucose levels rise in blood and tissue fluid, insulin stimulates the liver, fat, muscle and other tissues to take up glucose. Skeletal muscle is the largest tissues and organ of glucose consumption. The tolerance of muscle, liver and fat to insulin is the most important factor to induce metabolic disorders and type 2 diabetes mellitus. Macrophage infiltration is significantly increased in muscle and fat of obese or insulin-tolerant subjects. As obesity increases, macrophages accumulate between fat cells and muscle fibers. Type I macrophages can promote inflammation and secrete various factors such as TNF-α and IL-1β. TNF can stimulate the inflammatory signaling pathway in myocytes *in vitro,* which directly leads to the decreased sensitivity of insulin signaling pathway.

Skeletal muscle is highly plastic and regenerative. Understandably, its high plasticity is due to the fact that each skeletal muscle has more than 600 muscle fibers of different sizes and different contractile capacities. These muscle fibers work together to provide support for movement, postural maintenance, strength or fine movement, and even breathing. Satellite cells, i.e., muscle stem cells play a very important role in the repair function of skeletal muscle injury. Under normal conditions, satellite cells are in a resting state. Once they receive signals of injury or growth, satellite cells are rapidly activated and extensively migrate to the injured area to proliferate, differentiate and fuse into new myofiber cells. After repair of muscle injury, some satellite cells resist differentiation and return to the resting state and return to the nest. The important point is that the fate of satellite cells is greatly influenced by both endogenous and exogenous factors. The activation of satellite cells is related to inflammatory cells, stromal cells, nutrient signals and extracellular matrix composition in the environment.

With the continuous improvement of people's living standards, chronic diseases caused by diets high in fat, sugar and salt are increasing. Diabetes is one of the diseases that seriously affect human life and health, and has seriously threatened the quality of life of the global population. At present, the number of adult diabetic patients in China has reached 92.4 million, ranking first in the world. In type 2 diabetic subjects, adipose-infiltrated muscle tissue inflammation caused by obesity is enhanced, which on the one hand producing tolerance to insulin and on the other hand having an activating effect on satellite cells in the resting state.

There is an urgent need to develop effective drugs for type 2 diabetes mellitus.

### Summary of the invention

The purpose of the present invention is to provide a muscle stem cell for use in the preparation of pharmaceutical compositions that enhance muscle content and function and prevent, alleviate and/or treat metabolic disorders such as type 2 diabetes mellitus.

In the first aspect of the present invention, it provides a use of muscle stem cells for the preparation of a pharmaceutical composition for one or more applications selected from the following groups:
(a) Reduction in body weight;
(b) Improvement of glucose tolerance and/or insulin tolerance;
(c) Reduction in blood sugar;
(d) Improvement of insulin sensitivity;
(e) Reduction in fat content;
(f) Reduction in adipocyte weight;
(g) Reduction in adipocyte volume;
(h) Reduction in total cholesterol;
(i) Differentially regulating the expression levels of genes related to fat expression;
(j) Improvement of the expression level of genes related to brown fat expression;
(k) Reduction in the expression levels of genes related to white adipose expression;
(l) Maintenance of adipose tissue homeostasis;
(m) Reduction in liver tissue weight;
(n) Reduction in fatty infiltration of liver tissue;
(o) Reduction in the extent of fatty liver disease;
(p) Reduction in the level of inflammation of the body;
(q) Reduction in the expression of TNF-α and IL-1β in the body;
(r) Improvement of the expression of IL-10 in the body;
(s) Improvement of body metabolism;
(t) Prevention, alleviation and/or treatment of obesity;
(u) Prevention, alleviation and/or treatment of diabetes;
(v) Prevention, alleviation and/or treatment of insulin resistance;
(w) Prevention, alleviation and/or treatment of metabolic disorders;
(x) Improvement of muscle support;
(y) Enhancing athletic ability.

In another preferred example, the drug is used on humans or animals.

In another preferred example, the reduction in fat content includes reduction in subcutaneous fat content, reduction in abdominal fat content, reduction in liver tissue weight, and reduction in body weight.

In another preferred example, the improvement of insulin tolerance is to increase the subject's sensitivity to insulin.

In another preferred example, the genes related to brown fat expression are selected from the following group: *ucp1*, *tbxc1*, *pgc1α*, *tmem26*, *prdm16*, *cidea*, *pgc1β*, *cpt1α*, *cpt1β.*

In another preferred example, the genes related to white fat expression are selected from the following group: *leptin*, *fabp4*, *ppary*, *c*/*ebpα*, *c*/*ebpβ*, *clebpy, glut4, fasn*, *adiponectin.*

In another preferred example, the muscle stem cells are selected from the following groups: murine-derived muscle stem cells, human-derived muscle stem cells, monkey-derived muscle stem cells, dog-derived muscle stem cells, cat-derived muscle stem cells, horse-derived muscle stem cells, and a combination thereof.

In another preferred example, the mouse muscle stem cells are CD31⁻, CD34⁻, CD45⁻, Vcam1⁺, Intergrin-α7⁺ and PAX7⁺ cells.

In another preferred example, the human-derived muscle stem cells are CD31⁻, CD34⁻, CD45⁻, CD29⁺, EGFR⁺ and PAX7⁺ cells.

In another preferred example, other muscle stem cells of non-rodent and non-human origin are CD31⁻, CD34⁻, CD45⁻, CD29⁺, EGFR⁺ and PAX7⁺ cells.

In another preferred example, the metabolic disorders are selected from the following groups: diabetes mellitus, fatty liver disease, hypercholesterolemia, insulin resistance disease, hyperglycemia disease.

In another preferred example, the diabetes mellitus is selected from the following groups: type 1 diabetes mellitus, type 2 diabetes mellitus.

In another preferred example, the muscle stem cells are stem cells that can differentiate into striated muscle cells and have some characteristics of stellate cells.

In another preferred example, the pharmaceutical composition is used for the prevention and/or treatment of low muscle support and inadequate exercise capacity.

In another preferred example, the muscle stem cells are autologous, allogeneic, or xenogeneic.

In another preferred example, the muscle stem cells were selected from the following parts: limb skeletal muscle, trunk skeletal muscle.

In another preferred example, the dosage form of the pharmaceutical composition is selected from the following group: freshly cultured muscle stem cell injection, or thawed muscle stem cell injection from frozen storage.

In another preferred example, the muscle stem cells are extracted from the body muscle and obtained by cell sorting and expansion.

In another preferred example, the muscle stem cells are extracted from the limbs or trunk muscles of the body and obtained by cell sorting.

In another preferred example, the muscle stem cells are muscle-derived stem cells that can differentiate into striated muscle cells and have the characteristics of stellate cells.

In another preferred example, the dosage form of the pharmaceutical composition is an injection.

In another preferred example, the improvement of body metabolism includes: improvement of body glucose metabolism, improvement of body fat metabolism, improvement of body blood lipid metabolism, and a combination thereof.

In the second aspect of the present invention, it provides a pharmaceutical composition for human or animal comprising:
(i) a muscle stem cell as a first active ingredient; and
(ii) a pharmaceutically acceptable carrier;
the pharmaceutical composition is used for one or more applications selected from the following groups:
(a) Reduction in body weight;
(b) Improvement of glucose tolerance and/or insulin tolerance;
(c) Reduction in blood sugar;
(d) Improvement of insulin sensitivity;
(e) Reduction in fat content;
(f) Reduction in adipocyte weight;
(g) Reduction in adipocyte volume;
(h) Reduction in total cholesterol;
(i) Differentially regulating the expression levels of genes related to fat expression;
(j) Improvement of the expression level of genes related to brown fat expression;
(k) Reduction in the expression levels of genes related to white adipose expression;
(l) Maintenance of adipose tissue homeostasis;
(m) Reduction in liver tissue weight;
(n) Reduction in fatty infiltration of liver tissue;
(o) Reduction in the extent of fatty liver disease;
(p) Reduction in the level of inflammation of the body;
(q) Reduction in the expression of TNF-α and IL-1β in the body;
(r) Improvement of the expression of IL-10 in the body;
(s) Improvement of body metabolism;
(t) Prevention, alleviation and/or treatment of obesity;
(u) Prevention, alleviation and/or treatment of diabetes;
(v) Prevention, alleviation and/or treatment of insulin resistance;
(w) Prevention, alleviation and/or treatment of metabolic disorders;
(x) Improvement of muscle support;
(y) Enhancing athletic ability.

In another preferred example, the pharmaceutical composition also comprises a second active ingredient that regulates glucose metabolism, preferably, the second active ingredient selected from the following group:
biguanides, sulfonylureas, non-sulfonylureas insulin secretagogues, thiazolidinedione insulin sensitizers, glycosidase inhibitors, insulin, glucagon-like peptide-1 analogues or agonists.

In another preferred example, the muscle stem cell is a mouse muscle stem cell, preferably, CD31⁻, CD34⁻, CD45⁻, Vcam1⁺, Intergrin-α7⁺ and PAX7⁺ cell.

In another preferred example, the muscle stem cell, preferably, the human-derived muscle stem cell is CD31⁻, CD34⁻, CD45⁻, CD29⁺, EGFR⁺ and PAX7⁺ cell.

In another preferred example, the muscle stem cell is non-rodent derived muscle stem cell, preferably, CD31⁻, CD34⁻, CD45⁻, CD29⁺, EGFR⁺ and PAX7⁺ cell.

In another preferred example, the pharmaceutical composition is an anti-type 1 diabetes mellitus drug.

In another preferred example, the pharmaceutical composition is an anti-type 2 diabetes mellitus drug.

In another preferred example, the pharmaceutical composition is a drug that improves the body's glucose metabolism.

In another preferred example, the pharmaceutical composition is a drug for the prevention and/or treatment of muscle loss and strength deficiency.

In another preferred example, the pharmaceutical composition is a drug that maintains the homeostasis of the body's white adipose tissue.

In another preferred example, the pharmaceutical composition is a drug that reduces the inflammatory environment of the body.

In another preferred example, the pharmaceutical composition is a drug that reduces the extent of fatty liver disease.

In another preferred example, the pharmaceutical composition is for the prevention and/or treatment of low muscle support.

In another preferred example, the pharmaceutical composition is for the prevention and/or treatment of insufficient athletic ability.

In another preferred example, the dosage form of the pharmaceutical composition is selected from the following group: fresh or frozen muscle stem cell injection, lyophilized agent.

In the third aspect of the invention, it provides a method of prevention, alleviation and/or treatment of metabolic disorders, which comprises: administering to a subject in need thereof a muscle stem cell, collection, cryostorage and pharmaceutical compositions as the second aspect of the present invention, or a preparation comprising a muscle stem cell or a pharmaceutical composition, and a combination thereof.

It is to be understood that the various technical features of the present invention mentioned above and the various technical features specifically described hereinafter (as in the Examples) may be combined with each other within the scope of the present invention to constitute a new or preferred technical solution, which needs not be described one by one, due to space limitations.

### Description of Drawings

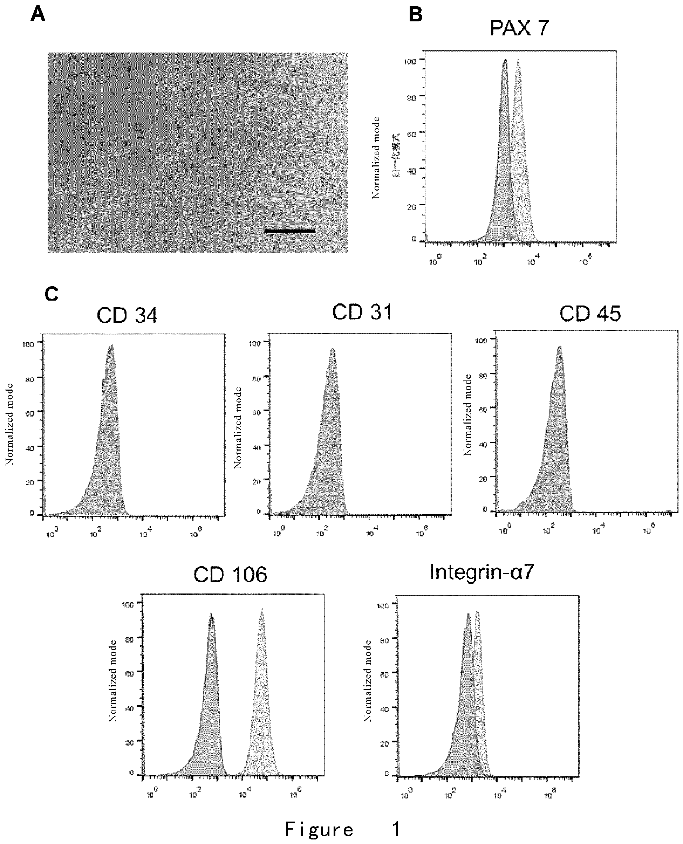

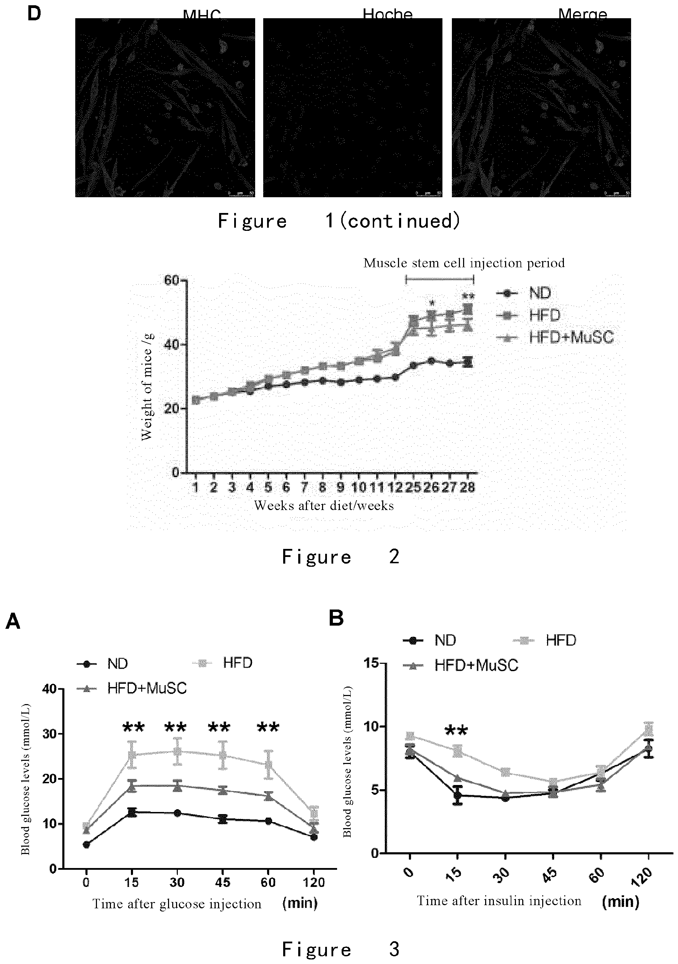

Figure 1 shows A. Morphology of murine derived muscle stem cells, which are spindle shaped. Scale bar = 500 µm; B. Flow cytometry was used to analyse PAX7 protein specifically expressed in mouse muscle stem cells; C. Identification of mouse muscle stem cells by surface marker staining. Cell surface markers CD31-APC, CD34-PerCP-cy5.5, CD45-APC, CD106(Vcam-1)-V450 and Intergrin-α7-FITC were used for staining and identification; D. Immunofluorescence staining for identification of myosinogen heavy chain (MHC), a specific differentiated mature protein of mouse muscle stem cells. Red is myosin heavy chain, blue is nucleus, wherein MHC staining is myosin heavy chain staining and Hochest staining is nuclear staining. Merge is a overlap graph. Scale bar = 50 µm.

Figure 2 shows the trend curve of weight gain of mice. ND was a normal diet mice group and mice were fed with 10% Kcal of normal diet, HFD was a high-fat diet mice group and mice were fed with 60% Kcal of high-fat diet, and HFD + MuSC was a group of mice injected to high-fat diet mice with mouse muscle stem cells. At week 12, mice were injected with muscle stem cells.

Figure 3 shows A. The capacity in reduced blood glucose of mice was identified by glucose tolerance test. Mice were fasted for 15 hours and fasting blood glucose was measured. Glucose was intraperitoneally injected into the mice at the ratio of 1g/Kg mice, and the blood glucose values of the mice were detected at 15 min, 30 min, 45 min, 60 min and 120 min after injection to form a blood glucose curve. B. The capacity in reduced blood glucose of mice was tested by insulin tolerance test. The blood glucose of each group was measured. Insulin was intraperitoneally injected into the mice at the ratio of 0.75 U/Kg mice, and the blood glucose values of the mice were measured at 15 min, 30 min, 45 min, 60 min, and 120 min after injection to form a blood glucose curve.

Wherein ND was a normal diet mice group and mice were fed with 10% Kcal of normal diet, HFD was a high-fat diet mice group and mice were fed with 60% Kcal of high-fat diet, and HFD + MuSC was a group of mice injected to high-fat diet mice with mouse muscle stem cells.

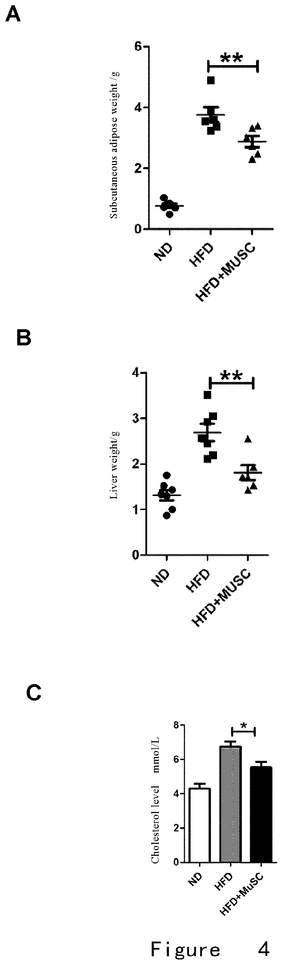

Figure 4 shows A. Subcutaneous adipose tissue was obtained from mice of each group and weighed for comparison; B. The liver tissues of mice in each group were obtained and weighed for comparison; C. Total cholesterol in peripheral blood serum of mice in each group was detected and compared by kit;

Wherein ND was a normal diet mice group and mice were fed with 10% Kcal of normal diet, HFD was a high-fat diet mice group and mice were fed with 60% Kcal of high-fat diet, and HFD + MuSC was a group of mice injected to high-fat diet mice with mouse muscle stem cells.

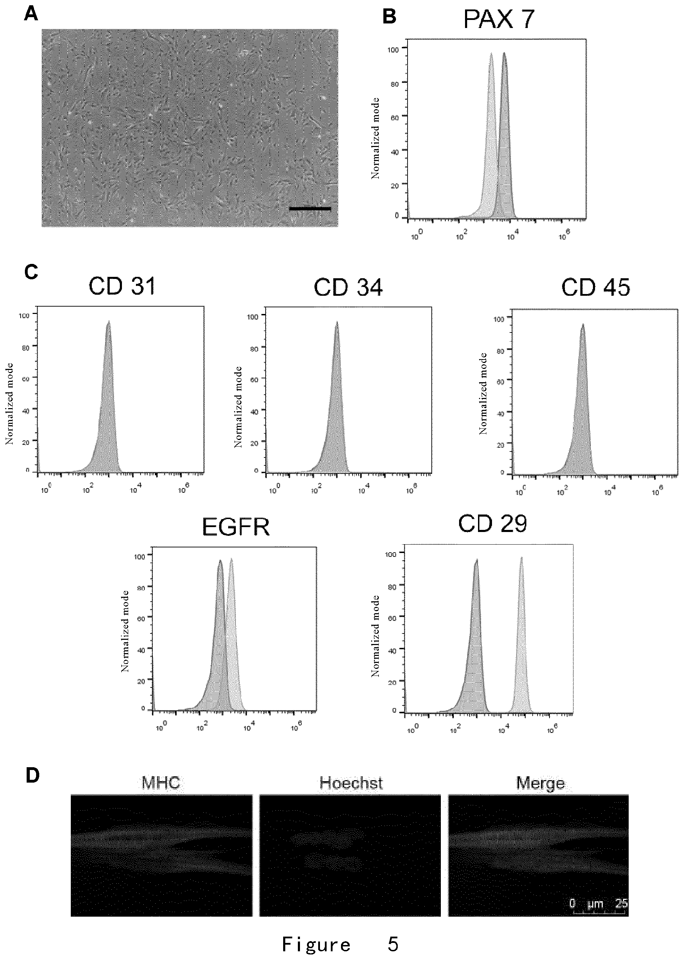

Figure 5 shows A. Human-derived muscle stem cells are spindle-shaped. Scale bar =500µm; B. Flow cytometric identification of specific nuclear factor PAX7 expressed by human-derived muscle stem cells. PAX7 protein antibody was used to stain muscle stem cells after membrane rupture and fixation. Fluorescence secondary antibody with excitation wavelength of 647 was used to stain muscle stem cells, and flow cytometry analyzer was used to analyze cells. C. Flow cytometric identification of surface markers of human-derived muscle stem cells expression. The surface markers of isolated muscle stem cells were stained with anti-mouse flow cytometric antibodies CD31-PE, CD34-PE, CD45-PE, CD29-APC and EGFR-BV421, and analyzed by flow cytometry. D. Immunofluorescence staining for identification of myogen heavy chain (MyHC), which is a mature protein specific differentiated by human-derived muscle stem cells, and red is myosin heavy chain, blue is nucleus. Scale bar = 25 µm;

Wherein MHC staining was myosin heavy chain staining and Hochest staining was nuclear staining. Merge is a overlap graph.

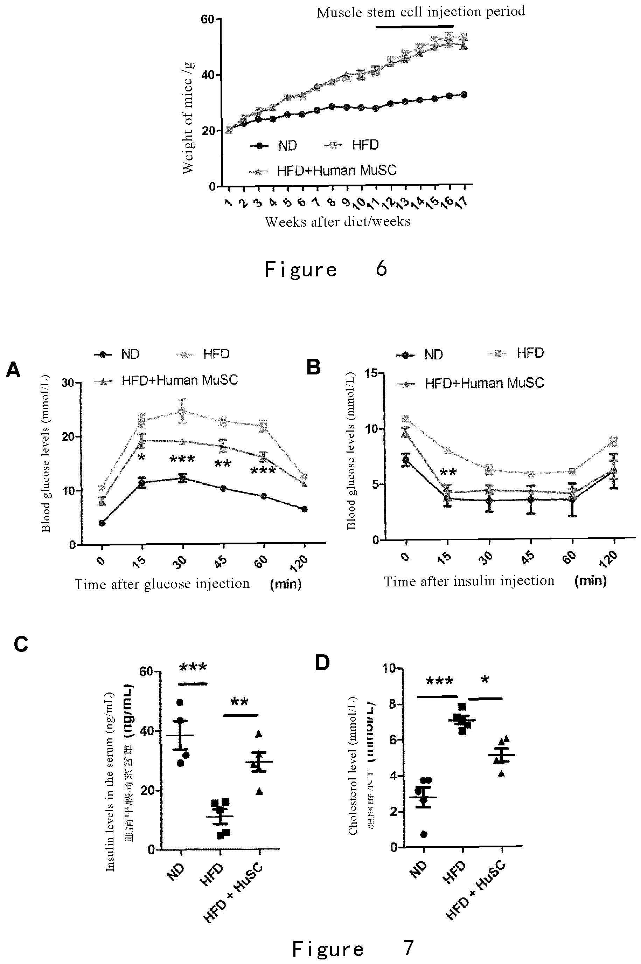

Figure 6 shows the trend curve of weight gain of mice, wherein ND was the normal diet mice group, fed with 10% Kcal of normal diet, HFD was the high-fat diet mice group, fed with 60% Kcal of high-fat diet. HFD + Human MuSC was the group of mice injected with human-derived muscle stem cells in high-fat diet mice, and the weight of high-fat diet mice was significantly increased. After 2 months of high-fat diet, exogenous administration of human-derived muscle stem cells significantly inhibited weight gain in mice.

Figure 7 shows A. Glucose tolerance test was performed to detect the capacity in reduced blood glucose of mice. Fasting blood glucose was measured after mice in each group were fasted for 15 hours. Glucose was intraperitoneally injected into the mice at the ratio of 1g/Kg mice, and the blood glucose values of the mice were detected at 15 min, 30 min, 45 min, 60 min and 120 min after injection to form a blood glucose curve. B. Insulin tolerance test was used to detect the capacity in reduced blood glucose of mice. The blood glucose of mice in each group was measured. Insulin was intraperitoneally injected into the mice at the ratio of 0.75 U/Kg mice, and the blood glucose values of the mice were measured at 15 min, 30 min, 45 min, 60 min, and 120 min after injection to form a blood glucose curve. C. The expression of insulin in mouse serum was detected by insulin ELISA kit. D. Total cholesterol (T-CHO) detection kit was used to detect the content of total cholesterol in serum of mice;

Wherein ND was the normal diet mice group and mice were fed with 10% Kcal of normal diet, HFD was the high-fat diet mice group and mice were fed with 60% Kcal of high-fat diet, and HFD + Human MuSC (HFD + HuSC) was a group of mice injected to high-fat diet mice with human-derived muscle stem cells.

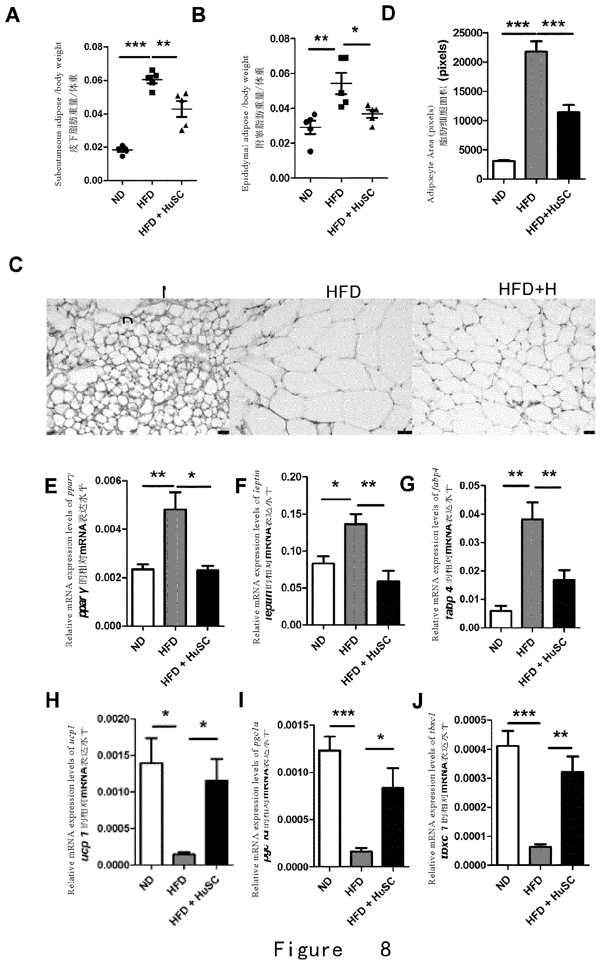

Figure 8 shows A. Abdominal subcutaneous fat was obtained from mice in each group, and the subcutaneous adipose tissue was weighed; B. Determination of adipose tissue content in abdominal cavity and epididymis of mice; C, D. Comparison of adipocyte volume and statistics in mouse adipose tissue sections. The adipose tissue was dehydrated and fixed before paraffin embedded sectioning. The sections were stained with hematoxylin and eosin. Scale bar = 75 µm; E, F, G. QPCR was used to detect the expression of white adipose-related genes *ppar Y, leptin* and *fabp4* in mouse adipose tissue. H, I, J. QPCR was used to detect the expression of brown adipose-related genes *ucp 1*, *tbxc1* and *pgc1a* in mouse adipose tissue.

Wherein ND was the normal diet mice group and mice were fed with 10% Kcal of normal diet, HFD was the high-fat diet mice group and mice were fed with 60% Kcal of high-fat diet, and HFD + HuSC was a group of mice injected to high-fat diet mice with human-derived muscle stem cells.

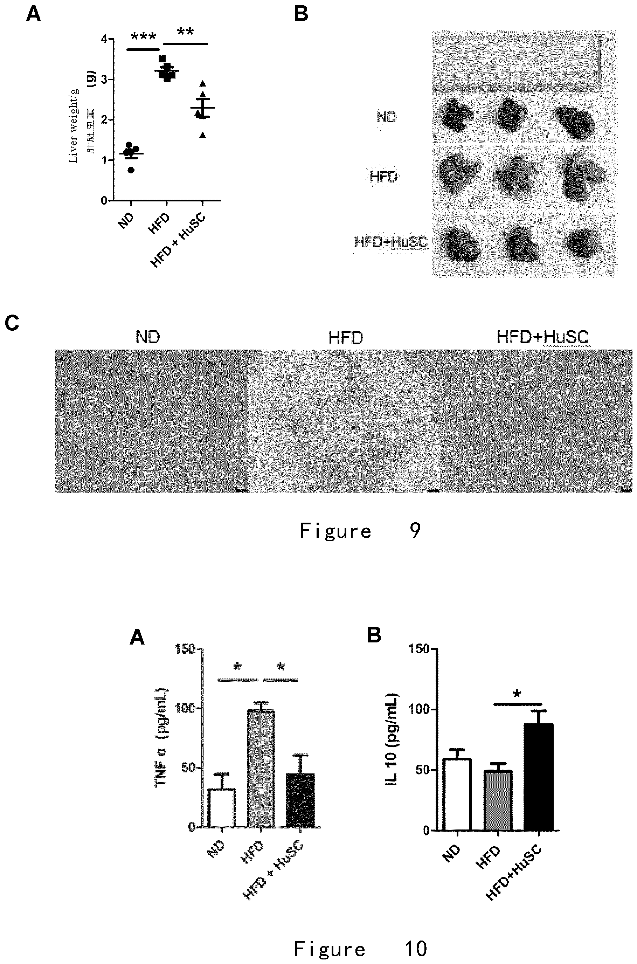

Figure 9 shows A. The liver tissues of mice in each group were obtained, and the liver tissues of mice were weighed and compared; B. The obtained liver tissue was photographed to compare the appearance of liver tissue; C. Hematoxylin-eosin staining was performed after paraffin-embedded sections of liver tissue, and the degree of fatty liver diseases was compared between the stained sections. Scale bar = 75 µm;
wherein, ND was the normal diet mice group and mice were fed with 10% Kcal of normal diet, HFD was the high-fat diet mice group and mice were fed with 60% Kcal of high-fat diet, and HFD + HuSC was a group of mice injected to high-fat diet mice with human-derived muscle stem cells.

Figure 10 shows A. The expression level of TNF-α in serum of mice in each group was detected by ELISA kit of TNF-α and compared. B. The content of IL-10 in serum of mice in each group was used to detect by IL-10 ELISA kit and compared;
wherein, ND was the normal diet mice group and mice were fed with 10% Kcal of normal diet, HFD was the high-fat diet mice group and mice were fed with 60% Kcal of high-fat diet, and HFD + HuSC was a group of mice injected to high-fat diet mice with human-derived muscle stem cells.

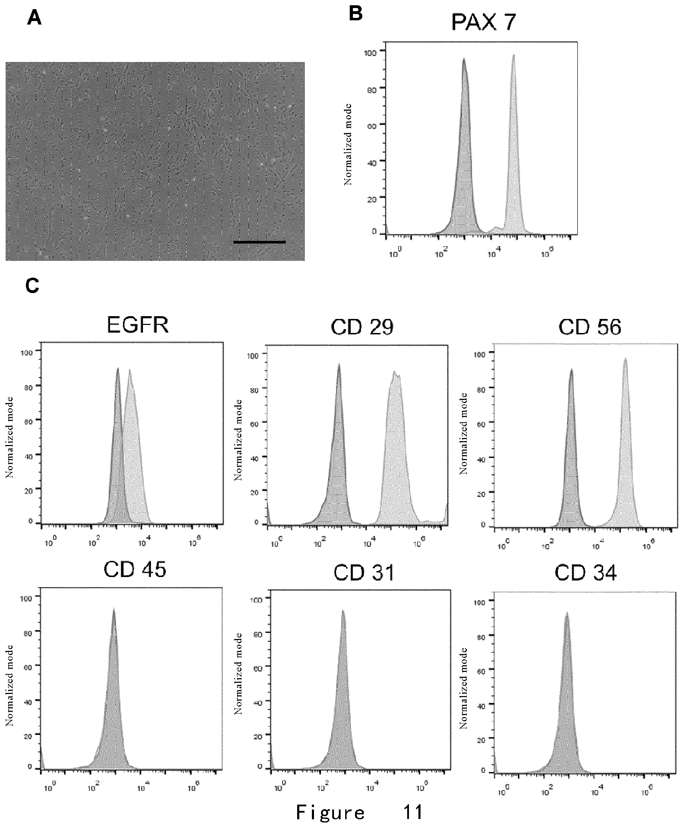

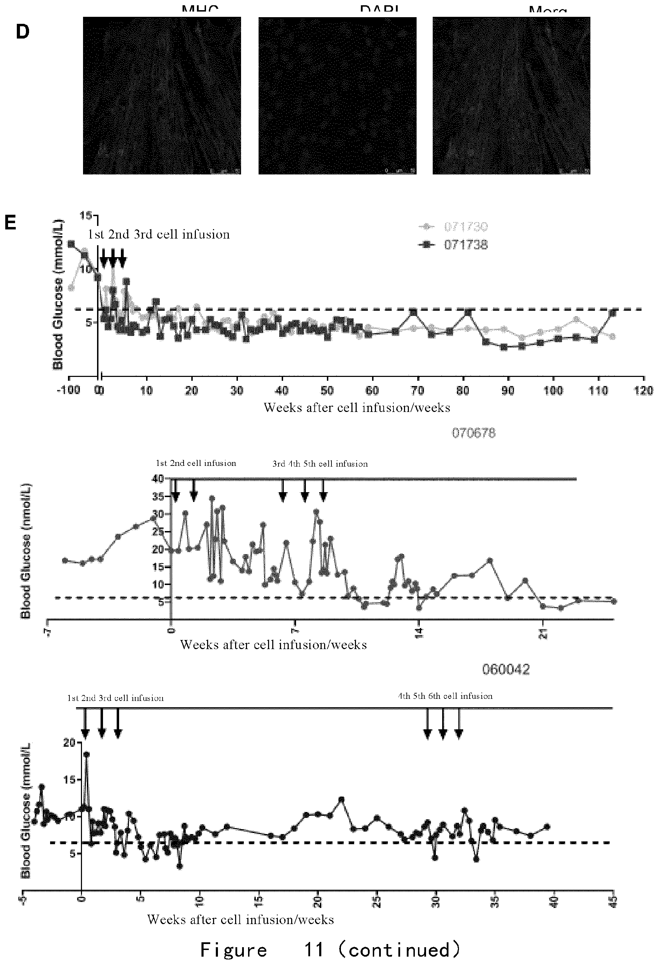

Figure 11 shows A. spindle-shaped morphology of monkey derived muscle stem cells; B. Identification of muscle stem cell-specific nuclear factor PAX7 staining for monkey-derived muscle stem cells. C. Identification of cell surface marker proteins in monkey derived muscle stem cells. CD31-PE, CD34-PE, CD45-PE,CD29-APC,CD56-V450 and EGFR-V450 flow cytometric antibody were used for flow cytometric identification of muscle stem cells staining. D. Immunofluorescence staining for identification of specific differentiated mature protein myogen heavy chain (MyHC) of monkey derived muscle stem cells (red is myosin heavy chain, blue is nucleus). Scale bar = 50 µm; E. Changes in fasting blood glucose values of 4 monkeys (experimental monkey numbers: 071730,071738,070678 and 060042) before and after intravenous infusion of autologous muscle stem cells in monkeys with spontaneous hyperglycemia. The cell injection volume was 2.5×10⁶ cells /Kg experimental monkey, the cell injection cycle was once every 2 weeks, a total of 3-6 times;

Wherein MHC staining was myosin heavy chain staining, Hochest staining was nuclear staining. Merge is a overlap graph.

In all above results, **P*<0.05; **P<0.01; ****P*<0.001.

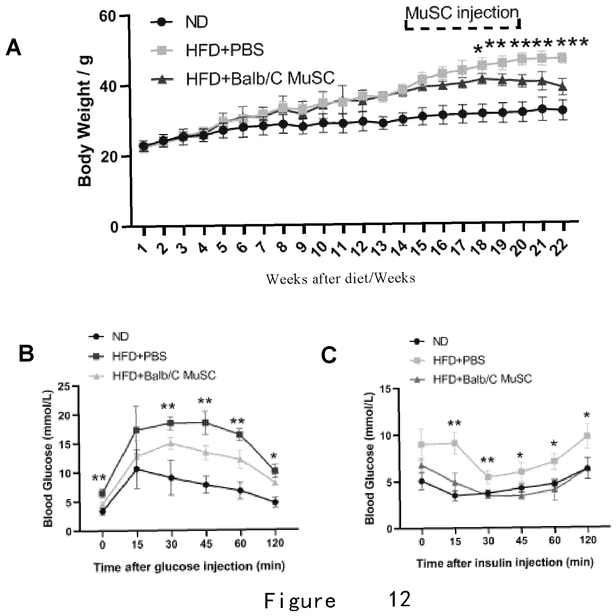

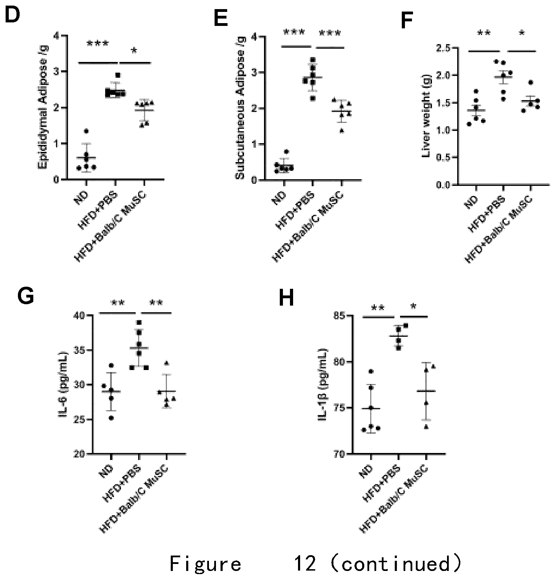

Figure 12 shows A. Trend curve of weight gain of mice. B The hypoglycemic ability of mice was evaluated by glucose tolerance test. Mice were fasted for 15 hours and fasting blood glucose was measured. Glucose was intraperitoneally injected into the mice at the ratio of 1g/Kg mice, and the blood glucose values of the mice were measured at 15min, 30min, 45min, 60min and 120min after injection to form a blood glucose curve. C. The hypoglycemic ability of mice was tested by insulin tolerance test. The blood glucose of each group was measured. Insulin was intraperitoneally injected into the mice at the ratio of 0.75U/Kg mice, and the blood glucose values of the mice were detected at 15min, 30min, 45min, 60min and 120min after injection to form a blood glucose curve. D. Determination of adipose tissue content in abdominal cavity and epididymis of mice; E. Abdominal subcutaneous fat of mice in each group was obtained, and the subcutaneous adipose tissue of mice was weighed; F. Mouse liver tissue was weighed and compared; G. The expression level of TNF-α in serum of each group was detected by IL-6 ELISA kit and compared. H. The expression level of IL-10 in serum of each group was detected by IL-1β ELISA kit and compared.

Wherein ND mice were fed with 10% Kcal of normal diet, HFD mice were fed with 60% Kcal of high fat diet, and HFD + MuSC mice were a group of mice injected to high-fat diet mice with Balb/C murine derived muscle stem cells. At week 14, mice were injected with muscle stem cells.

In all above results, **P*<0.05; **P<0.01; ****P*<0.001.

### Modes for Carrying Out the Present Invention

Through extensive and intensive research, the inventors have unexpectedly discovered that activated satellite cells have an inhibitory effect on the inflammatory response caused by obesity, thereby reducing the level of inflammation, enhancing the sensitivity of tissues to insulin, regulating the level of tissue metabolism, reducing blood glucose, reducing fat enlargement and infiltration, and playing a therapeutic role in type 2 diabetes mellitus.

Specifically, a mouse model of insulin tolerance induced by a high-fat diet is selected in the present invention to investigate the use of human derived muscle stem cells in the preparation of pharmaceutical compositions for the prevention, remission and/or treatment of metabolic disorders (such as type 2 diabetes mellitus). Human cells injected into mice can be eliminated by the body in a short period of time, which excludes the possibility of cell colonization, proliferation and differentiation in the subject.

Muscle stem cells were injected into the tail vein of insulin tolerant mice induced by high-fat diet in the present invention. The experimental results show that compared with model group, the treatment group can significantly improve the glucose metabolism ability of mice, reduce the white adipose tissue, alleviate the enlargement of adipocytes, improve the white adipose tissue homeostasis, alleviate the inflammatory environment of mice, and reduce the extent of fatty liver disease. The present invention has been completed on the basis of these studies.

### Term

Unless otherwise defined, all technical and scientific terms used herein have the same meaning as would normally be understood by ordinary technicians in the field to which the invention belongs. As used in this application, when referring to a specific enumerated value, the term "approximately" means that the value can vary by no more than 1% from the enumerated value. For example, as used in this application, the statement "about 100" includes all values between 99 and 101 and (for example, 99.1, 99.2, 99.3, 99.4, and so on).

Although any method and material similar or equivalent to those described in the present invention may be used in the implementation or testing of the present invention, the preferred method and material is presented here.

### Muscle stem cell

Mauro et al. first proposed the discovery of muscle satellite cells, muscle stem cell precursors, in frog skeletal muscle in 1961. Subsequently, more and more studies have proved that there are a few muscle stem cells in adult mammalian skeletal muscle, and the number of muscle stem cells gradually decreases with age.

Muscle satellite cells are myogenic stem cells located between the basement membrane of skeletal muscle and the membrane of muscle fibers. In physiological condition, muscle satellite cells exist in stationary undifferentiated state, after the damage or inflammation stimulation activated muscle stem cells to divide for the spindle muscle stem cells, and along with the increasing division multicore muscular tube mutual fusion, finally developed to form raw muscle fibers or incorporate into the damaged muscle tissue. It plays an important role in skeletal muscle growth, injury repair, function maintenance and tissue regeneration.

In a preferred example of the invention, the muscle stem cells are selected from the following groups: murine muscle stem cells, human-derived muscle stem cells, monkey muscle stem cells, dog muscle stem cells, cat muscle stem cells, horse muscle stem cells, etc.

In a preferred example of the invention, the mouse muscle stem cells are cells with CD31⁻, CD34⁻, CD45⁻, Vcam1⁺, Intergrin-α7⁺ and PAX7⁺.

In a preferred example of the invention, the human-derived muscle stem cells are cells with CD31⁻, CD34⁻, CD45⁻, CD29⁺, EGFR⁺ and PAX7⁺.

Other muscle stem cells of non-rodent and non-human origin are cells with CD31⁻, CD34⁻, CD45⁻, CD29⁺, EGFR⁺ and PAX7⁺.

In a preferred embodiment of the present invention, said muscle stem cells are muscle-derived, differentiable into striated muscle cells with some of the properties of stellate cells.

In a preferred example of the invention, the muscle stem cells are autologous, allogeneic, or xenogeneic.

In a preferred example of the invention, the muscle stem cells were selected from the following parts: limb skeletal muscle, trunk skeletal muscle.

In a preferred example of the invention, the muscle stem cells are extracted from the body muscle and obtained by cell sorting and expansion.

In a preferred example of the invention, the muscle stem cells are extracted from the limbs or trunk muscles of the body and obtained by cell sorting.

### Muscle-derived mesenchymal stem cells

Mesenchymal stem cells are found in both skeletal and smooth muscle. The main role of muscle injury repair and regeneration is muscle stem cells, but some other groups of cells, such as inflammatory cells, vascular endothelial cells and muscle-derived mesenchymal stem cells, play a fine regulatory role in the whole process of muscle injury repair and regeneration. For example, fibroblast adipose progenitor cells are a kind of mesenchymal stem cells in the muscle stroma, whose role is to support the differentiation of muscle stem cells and promote the repair and regeneration of muscle tissue. The main role of smooth muscle-derived muscle stem cells in the blood vessels is to replace smooth muscle cells, so as to fill the area of vascular lesions or injuries.

### Metabolic disorder disease

Metabolic disorder is a state of the body, is the body's absorption, digestion, excretion of substances appear pathological disorders, resulting in imbalance between supply and demand. It can be a disorder of one substance or many substances. The various metabolic state disorders vary differently. Glucose metabolism disorder causes diabetes, lipid metabolism disorder causes hyperlipidemia, uric acid metabolism disorder causes gout and so on. Electrolyte also can appear metabolic disorder, cause corresponding disorder state, such as high potassium, hypokalemia and so on. Metabolic diseases characterized by chronic hyperglycemia are often associated with lipid metabolism disorder in clinic, which has become one of the main complications of diabetes mellitus. The direct symptom of lipid metabolism disorder is the increase of total cholesterol index.

In a preferred example of the invention, the metabolic disorders are selected from the following groups: diabetes mellitus, fatty liver disease, hypercholesterolemia, insulin resistance disease, hyperglycemia disease.

In another preferred example of the invention, the diabetes mellitus is selected from the following group: type 1 diabetes mellitus, type 2 diabetes mellitus.

### Pharmaceutical composition

The invention also provides a composition. In the preferred example, the composition is a pharmaceutical composition comprising the aforementioned muscle stem cells and a pharmaceutically acceptable carrier. In general, these substances may be formulated in a non-toxic, inert and pharmaceutically acceptable aqueous carrier medium, wherein the pH is generally about 5-8, preferably, pH is about 6-8, although the pH value may be varied depending on the nature of the substances to be formulated and the condition to be treated. The formulated pharmaceutical composition may be administered by conventional routes, including (but not limited to): oral, respiratory, intratumoral, intraperitoneal, intravenous, or topical administration.

The pharmaceutical composition of the invention may be used directly for the treatment (for example, for the treatment of metabolic disorders). In addition, other therapeutic agents can also be used simultaneously.

The pharmaceutical composition according to the present invention comprises a safe and effective amount (e.g. 0.001-99 wt%, preferably 0.01-90 wt%, preferably 0.1-80 wt%) of the muscle stem cells according to the present invention and a pharmaceutically acceptable carrier or excipient. Such carriers include, but are not limited to, saline, buffer solution, glucose, water, glycerin, ethanol, dimethyl sulfoxide (DMSO) or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 microgram per kilogram body weight to about 10 milligrams per kilogram body weight per day. Further, the muscle stem cells of the present invention can also be used in combination with the other therapeutic agents.

When a pharmaceutical composition is used, a safe and effective amount of the muscle stem cells is administered to a mammal, wherein the safe and effective amount is usually at least about 10 µg/Kg body weight, and in most cases does not exceed about 8 mg/kg body weight, preferably the dose is about 10 µg/Kg body weight to about 1 mg/kg body weight. Of course, the particular dose should also depend on various factors, such as the route of administration, patient healthy status, which are well within the skills of an experienced physician.

In a preferred example of the invention, the pharmaceutical composition comprising:
(i) muscle stem cells as a first active ingredient; and
(ii) a pharmaceutically acceptable carrier;
the pharmaceutical composition is used for one or more applications selected from the following groups:
(a) Reduction in body weight;
(b) Improvement of glucose tolerance and/or insulin tolerance;
(c) Reduction in blood sugar;
(d) Improvement of insulin sensitivity;
(e) Reduction in fat content;
(f) Reduction in adipocyte weight;
(g) Reduction in adipocyte volume;
(h) Reduction in total cholesterol;
(i) Differentially regulating the expression levels of genes related to fat expression;
(j) Improvement of the expression level of genes related to brown fat expression;
(k) Reduction in the expression levels of genes related to white adipose expression;
(l) Maintenance of adipose tissue homeostasis;
(m) Reduction in liver tissue weight;
(n) Reduction in fatty infiltration of liver tissue;
(o) Reduction in the extent of fatty liver disease;
(p) Reduction in the level of inflammation of the body;
(q) Reduction in the expression of TNF-α and IL-1β in the body;
(r) Improvement of the expression of IL-10 in the body;
(s) Improvement of body metabolism;
(t) Prevention, alleviation and/or treatment of obesity;
(u) Prevention, alleviation and/or treatment of diabetes;
(v) Prevention, alleviation and/or treatment of insulin resistance;
(w) Prevention, alleviation and/or treatment of metabolic disorders;
(x) Improvement of muscle support;
(y) Enhancing athletic ability.

In a preferred example of the invention, the mouse muscle stem cells are cells with CD31⁻, CD34⁻, CD45⁻, Vcam1⁺, Intergrin-α7⁺ and PAX7⁺.

In another preferred example, the human-derived muscle stem cells are cells with CD31⁻, CD34⁻, CD45⁻, CD29⁺, EGFR⁺ and PAX7⁺.

Other muscle stem cells of non-rodent and non-human origin are cells with CD31⁻, CD34⁻, CD45⁻, CD29⁺, EGFR⁺ and PAX7⁺.

In a preferred example of the invention, the pharmaceutical composition is an anti-type 1 diabetes mellitus drug.

In a preferred example of the invention, the pharmaceutical composition is an anti-type 2 diabetes mellitus drug.

In a preferred example of the invention, the pharmaceutical composition is a drug that improves the body's glucose metabolism.

In another preferred example, the pharmaceutical composition is for the prevention and/or treatment of low muscle support.

In another preferred example, the pharmaceutical composition is for the prevention and/or treatment of insufficient athletic ability.

In a preferred example of the invention, the pharmaceutical composition is a drug that maintains the homeostasis of the body's white adipose tissue.

In a preferred example of the invention, the pharmaceutical composition is a drug that reduces the inflammatory environment of the body.

In a preferred example of the invention, the pharmaceutical composition is a drug that reduces the extent of fatty liver disease.

In a preferred example of the invention, the dosage form of the drug composition is selected from the following groups: injection and lyophilized agent.

In a preferred example of the invention, the dosage form of the anti-type 2 diabetes mellitus drug is selected from the following group: freshly cultured muscle stem cell injection, or thawed muscle stem cell injection from frozen storage.

In a preferred example of the invention, the anti-type 2 diabetes drug can significantly improve the glucose metabolism ability of mice and reduce blood glucose.

In a preferred example of the invention, the anti-type 2 diabetes drug is able to reduce white adipose tissue weight, relieve adipocyte hypertrophy, and improve white adipose tissue homeostasis.

In a preferred example of the invention, the anti-type 2 diabetes drug is able to reduce the level of inflammation in mice.

In a preferred example of the invention, the anti-type 2 diabetes drug is able to reduce the extent of fatty liver disease.

### The main advantages of the present invention include:

(1) By injecting muscle stem cells into tail vein of insulin tolerant mice induced by high-fat diet, the present invention fully studies the effect of muscle stem cells in the treatment of metabolic disorders;
(2) The invention unexpectedly discovers that muscle stem cells have the following functions:
   1. Improving the glucose metabolism ability of mice;
   2. Reducing fasting blood glucose;
   3. Reducing the amount of white fat, alleviating adipocyte hypertrophy, and maintaining adipose tissue homeostasis;
   4. Reducing the level of inflammation;
   5. Reducing the extent of liver fatty disease;
   6. Improving the body muscle state and other physiological functions;
(3) The invention can achieve relatively long-term therapeutic effect through a limited number of muscle stem cell injections, resulting in the following advantages:
   1. The number of cell injections is low, reducing the number of times the body suffers pain and improving the quality of the body's survival;
   2. A few injections of muscle stem cells can have a long-lasting effect with the advantages of saving time and effort.
(4) A single separation can be long-term frozen storage for multiple uses.

The present invention will be further illustrated below with reference to the specific examples. It is to be understood that these examples are for illustrative purposes only and are not intended to limit the scope of the invention. For the experimental methods in the following examples, in which the specific conditions are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless indicated otherwise, parts and percentage are weight parts and weight percentage. The experimental materials involved in the invention can be obtained from commercial channels without special instructions.

### Example 1: Extraction, expansion, identification and differentiation of mouse muscle stem cells

### (1) Extraction of murine derived muscle stem cells

1. Hind leg muscle tissue of C57/BL mice (C57/BL mice are used unless otherwise noted in the following) was cut into pieces to form tissue serosa, 10mL collagenase type II (750U/mL) was added, and was incubated at 37 °C for 60min at a constant temperature shaker with a rotational speed of 70rpm.
2. After incubation and digestion, complete medium (containing 10% FBS) was used for neutralization, and the supernatant was removed after centrifugation.
3. The mixed digestion solution of 10mL collagenase type II (100U/mL) and dispersive enzyme (1.1U/mL) was added to the precipitated cell pellet again, and the cell precipitation was resuspended.
4. The cell suspension was incubated again in a constant temperature shaker for digestion. The incubation condition was 37 °C for 60min, and the shaker speed was 70rpm.
5. At the end of incubation, neutralizing solution was added to terminate digestion, and the obtained liquid was centrifuged for 5min, and the supernatant was removed to obtain cell precipitation.
6. The cell precipitate was resuspended, and incubated with the mixed solution of monoclonal antibody labeled with magnetic beads in the mouse muscle satellite cell separation kit for 30min. Non-target cells would directly bind to the antibody. After cleaning with neutralizing solution, negative sorting of magnetic beads was carried out.
7. After magnetic bead sorting, cell complete medium (80% F12 medium, 20% fetal bovine serum, 1% P/S, 100ng/mL TNF-α, 100ng/mL IFN-γ, 50ng/mL IL-1α, 50ng/mL IL-13) was added. Then the mixture was transferred to petri dishes coated with collagen in advance and cultured in an incubator.

### (2) Expansion and identification of mouse muscle stem cells

1. When the cell proliferation reached 70% confusion, the cells were digested and passaged, and the cells were implanted in a new extracellular matrix (ECM) coated petri dish.
2. Flow cytometric antibodies CD31⁻-PE, CD34⁻-PE, CD45⁻-PE, Vcam1⁺-BV421 and Intergrin-α7⁺-BV421 were used to stain and identify mouse muscle stem cells. The expression of specific nuclear factor PAX 7 in mouse muscle stem cells was determined by intracellular staining and flow cytometry.

### (3) Differentiation of mouse muscle stem cells

1. The muscle stem cells were cultured on collagen-coated plates until the cells were completely fused.
2. Cells were washed three times with serum-free DMEM medium, and then the medium was replaced with muscle stem cell differentiation medium (DMEM medium + 2% horse serum) for further culture. On the first day of differentiation culture, the cells began to elongate and intercellular fusion occurred. On the 2nd to 3rd day, cell fusion reached the peak, and most of the cells had completed the differentiation process into myotubes. At this time, immunofluorescence staining was performed for myogen heavy chain (MyHC), which is a mature protein specific differentiated by human-derived muscle stem cells, thus, identification experiments were performed to determine that the isolated cells had the differentiation characteristics of stem cells. The result is shown in Figure 1.

The result is shown in Figure 1.

Figure 1 shows that the isolated cells showed high expression of the muscle stem cell marker PAX7. The isolated cells were endothelial cell marker CD31 negative cells, hematopoietic stem cell marker CD34 negative cells, and leukocyte marker CD45 negative cells. The cells highly expressed epidermal growth factor receptor Vcam 1(CD 106) and Intergrin-α7 up to 90%. Therefore, the isolated cells were CD31⁻, CD34⁻, CD45⁻, Vcam 1⁺ and Intergrin-α7⁺ muscle stem cells. Muscle stem cells cultured and expanded *in vitro* can well maintain the expression of stem cell surface markers and nuclear specific factor PAX7, and can differentiate into mature and functional muscle fiber structure, indicating that the muscle stem cells cultured in the above system have excellent sternness and muscle fiber generation ability.

### Example 2: A mouse model of insulin tolerance induced by high-fat diet and transplantation of mouse muscle stem cells

### (1) Establishment of mouse model

Male C57/BL mice aged 4 weeks were randomly selected and divided intogroups, which were fed a normal diet containing 10% Kal fat and a high-fat diet containing 60% Kal fat, respectively. The body weight of mice was recorded daily to form a curve of body weight change. After 2-3 months of modeling, mice weighing about 40 g were selected for the experiment. Insulin tolerance was developed in high-fat diet fed mice due to obesity and can be used as a model of type 2 diabetes.

### (2) Mouse derived muscle stem cell transplantation in vivo

1. After the establishment of the model, the mice were divided into three groups: control group (normal diet group), model group (high-fat diet group) and treatment group (high-fat diet mice group injected with mouse muscle stem cells). Then the experiment was ready to begin.
2. The control group and the model group were injected with 200µL of the injection system sterile PBS every week by tail vein injection. The treatment group was injected with mouse muscle stem cells, the injection volume was 1-5 × 10⁵ cells per mouse, and the injection system was 200µL. The injection cycle was once a week, and a total of 6 injections were given.

The result is shown in Figure 2.

Figure 2 shows that the rate of weight gain of mice on a high-fat diet was significantly higher than that of mice on a normal diet. After 12 weeks of modeling, the weight of the high-fat diet mice in the model group and the treatment group reached 40 g, and the weight of the mice increased further with continuous high-fat diet. Mice in the control group on a normal diet had an average weight of 25 g at 8 weeks, and reached a plateau when their weight rose to 35g with further feeding. After intervention with mouse muscle stem cells (MuSC), the mice in the treatment group lost significantly more weight than those in the model group.

### Example 3: Regulation of glucose metabolism by murine derived muscle stem cells

### (1) Detection of glucose tolerance (GTT)

1. Model mice in Example 2 were given a treatment of fasting without water abstinence 15 hours before the test.
2. Fasting blood glucose of mice was detected by Byino blood glucose meter and blood glucose test strip, which was recorded as G0.
3. Mice were intraperitoneally injected with glucose at a dose of 1g/g. Immediately after injection, the time at the moment was recorded as T0.

After 15 min(T15), 30 min(T30), 45 min(T45), 60 min(T60) and 120min(T120), the blood glucose of the mice was measured again and recorded as G15, G30, G45, G60 and G120, respectively.

### (2) Detection of insulin tolerance (ITT)

1. Fasting blood glucose of mice was detected by Byino blood glucose meter and blood glucose test strip, which was recorded as G0.
2. Mice were intraperitoneally injected with short-acting insulin at a dose of 0.1U/g mouse. Immediately after injection, the time at the moment was recorded as T0.
3. After 15 min(T15), 30 min(T30), 45 min(T45), 60 min(T60) and 120 min(T120), the blood glucose of the mice was measured again and recorded as G15, G30, G45, G60 and G120, respectively.

The hypoglycemic ability of mice was detected by glucose tolerance and insulin tolerance test, so as to evaluate the glucose metabolism level of mice.

The result is shown in Figure 3.

Figure 3 shows that injection of murine derived muscle stem cells through tail vein significantly reduced fasting blood glucose in mice fed a high-fat diet. After intraperitoneal injection of glucose solution, the blood glucose level of mice in the model group increased rapidly and remained high for a long time. However, the blood glucose level of mice in control group and treatment group increased slowly and decreased to normal level quickly. Along with the body self-metabolism, the rate of hypoglycemia in the treatment group was significantly higher than that in the model group, indicating that the treatment group had a better ability of hypoglycemia. After short-acting insulin injection, the blood glucose values of mice in the control and treatment groups decreased rapidly, indicating that the mice in the treatment group were more sensitive to insulin than the mice in the model group and could quickly respond to the hypoglycemic effect of insulin. In conclusion, human-derived muscle stem cells have a good improvement effect on glucose metabolism in mice.

### Example 4: Regulation of lipid metabolism by murine derived muscle stem cells

### (1) Detection of adipose tissue weight

The subcutaneous adipose tissue of the model mice in Example 2 of each group was separated, weighed and detected, and the weight of adipose tissue of each group was compared.

### (2) Weight weighing of liver tissue

In type 2 diabetes caused by obesity, the liver tissue was complicated with fatty infiltration, forming fatty liver. The liver tissue of mice was removed and weighed for detection, and the weight of liver tissue of each group was compared.

### (3) Detection of mouse metabolites

1. Peripheral blood of mice was collected and left for 30min before centrifugation. The centrifugation condition was set at 300g for 30min.
2. Total cholesterol detection kit was used to detect the total cholesterol content in mice.

The result is shown in Figure 4.

Figure 4 shows that murine derived muscle stem cells significantly reduced subcutaneous fat content and liver tissue weight in treated mice. Moreover, in the peripheral blood, human derived muscle stem cells reduced total cholesterol. Therefore, murine derived muscle stem cells can improve lipid metabolism and blood lipid metabolism in mice.

### Example 5 Extraction, expansion, identification and differentiation of human-derived muscle stem cells

### (1) Extraction of human-derived muscle stem cells

1. Human muscle tissue was obtained from the hospital, which was cut into pieces to form tissue serosa, and put into a 50mL centrifuge tube.
2. 10mL collagenase type II (750U/mL) was added to the tissue serosa and was placed in a constant temperature shaker and incubated at 37°C for 60min with a shaker rotation speed of 70rpm.
3. After incubation and digestion, complete medium (containing 10% FBS) was used for neutralization, and the supernatant was removed after centrifugation.
4. The mixed digestion solution of 10mL collagenase type II (100U/mL) and dispersive enzyme (1.1 U/mL) was added to the precipitated cell pellet again, and the cell precipitation was resuspended.
5. The cell suspension was incubated again in a constant temperature shaker for digestion. The incubation condition was 37°C for 60min, and the shaker speed was 70rpm.
6. At the end of incubation, 10mL of complete medium was added for neutralization and filtered through a 40µm cell screen.
7. After filtration, the obtained liquid was centrifuged at 500g for 5min, and the supernatant was removed to obtain cell precipitation.
8. Cell precipitation was resuspended with 600 µL complete medium, and flow sorting was carried out.
9. 10µL of cell suspension was taken into a flow tube containing 190µL complete medium for control.
10. The remaining cell suspension was stained with flow sorting antibody, and the labeled antibodies were CD31-PE, CD34-PE, CD45-PE, CD29-APC and EGFR-BV421 for 30min. After staining, the complete medium was washed and centrifuged, and finally cells were resuspended using 200µL of complete medium.
11. The sample was loaded and subjected to flow sorting.

### (2) Expansion and culture of human derived muscle stem cells

1. After all cell suspensions were sorted, cells were centrifuged at 250g for 5min and the supernatant of washing medium was removed, and muscle stem cell growth medium (20% FBS, 40% DMEM/LOW, 40% MCDB131, 1%P/S, 1% insulin transferrin selenium, 10µM P38 inhibitor) was added. After resuspended, cultures were transferred to extracellular Matrix coated petri dishes (Extra Cellular Matrix, ECM coated for 24 hours) for culture and amplification.
2. The fresh medium was changed every two days until the degree of cell confluence reaches 60 ~ 70%.
3. Muscle stem cells were passaged with trypsin, and then the progeny cells were grown in new ECM-coated petri dishes for further culture and expansion.

### (3) Identification of human-derived muscle stem cells

1. The cells were grown to a confluent degree of 70%, and the required cells could be obtained by trypsin digestion.
2. Flow cytometry was used to identify the marker proteins expressed on the surface of human-derived muscle stem cells, and the expression of human-derived muscle stem cell specific nuclear factor PAX7 was identified by flow cytometry.

Cryo-storage of human-derived muscle stem cells. The expanded muscle stem cells in (2) can be long-term stored in liquid nitrogen in cryogenic solution containing dimethyl sulfoxide (DMSO).

### (4) Identification of muscle differentiation ability of human-derived muscle stem cells

1. The muscle stem cells were cultured on collagen-coated plates until the cells were completely fused.
2. Cells were washed three times with serum-free DMEM medium, and then replaced with muscle stem cell differentiation medium (DMEM medium +2% horse serum) for further culture. On the first day of differentiation culture, the cells began to elongate and intercellular fusion occurred. On the 2nd to 3rd day, cell fusion reached the peak, and most of the cells had completed the differentiation process into myotubes. At this time, immunofluorescence staining was performed for myogen heavy chain (MyHC), which is the specific differentiation mature protein of human-derived muscle stem cells, so as to identify experiments to determine that the isolated cells have the differentiation characteristics of stem cells.

The result is shown in Figure 5.

Figure 5 shows that the cells isolated by the invention highly expressed the muscle stem cell marker PAX7. The isolated cells were endothelial cell marker CD31 negative cells, hematopoietic stem cell marker CD34 negative cells, and leukocyte marker CD45 negative cells. The isolated cells highly expressed the stem cell marker CD29, and the expression ratio was 100%. The cells highly expressed the epidermal growth factor receptor EGFR by 90%.. Therefore, the isolated cells were CD31⁻, CD34⁻, CD45⁻, CD29⁺, EGFR⁺ and PAX7⁺ muscle stem cells. Muscle stem cells cultured and expanded *in vitro* can well maintain the expression of stem cell surface markers and nuclear specific factor PAX7, and can differentiate into mature and functional muscle fiber structure, indicating that the muscle stem cells cultured in the above system have excellent sternness and muscle fiber generation ability.

### Example 6 Insulin tolerance model induced by high-fat diet and transplantation of human-derived muscle stem cells

### (1) Establishment of mouse model

Male C57/BL mice aged 4 weeks were randomly selected and divided into groups. The mice were fed a normal diet containing 10% Kal fat and a high-fat diet containing 60% Kal fat, respectively. The body weight of mice was recorded to form a body weight change curve. After 2-3 months of modeling, mice with a body weight of about 40 g were selected for grouping and experiment, and the body weight of each mouse was continuously recorded. Insulin tolerance was developed in high-fat diet mice due to obesity, and thus can be used as a human type 2 diabetes model.

### (2) Human derived muscle stem cell transplantation in vivo

1. After the establishment of the model, the mice were divided into three groups: control group (normal diet group), model group (high-fat diet group) and treatment group (high-fat diet mice group injected with human-derived muscle stem cells).
2. The control group and the model group were injected with 200µL of the injection system sterile PBS every week by tail vein injection. The treatment group was injected with human-derived muscle stem cells (Human MuSC, HuSC), the injection volume was 1-5 ×10⁵ cells per mouse, and the injection system was 200µL. The injection cycle was once a week for a total of 6 injections.

The result is shown in Figure 6.

Figure 6 shows that the rate of weight gain of mice on the high-fat diet in the model and treatment groups was significantly higher than that of mice on the normal diet in the control group. After 8 weeks of modeling, the weight of the high-fat diet mice in the model group and the treatment group reached about 40 g, and the weight of the mice increased continuously with the continuous high-fat diet. Mice in the control group on a normal diet had an average weight of 25 g after 8 weeks, and reached a plateau when their weight rose to 35 grams with further feeding.

### Example 7 Improvement of glucose metabolism in mice by human-derived muscle stem cells

### (1) Detection of glucose tolerance (GTT)

1. Model mice constructed in Example 6 were given a treatment of fasting without water abstinence 15 hours before the test.
2. Fasting blood glucose of mice was detected by Byino blood glucose meter and blood glucose test strip, which was recorded as G0.
3. Mice were intraperitoneally injected with glucose at a dose of 1g/g mice. Immediately after injection, the time at the moment was recorded as T0.
4. After 15 min(T15), 30 min(T30), 45 min(T45), 60 min(T60) and 120 min(T120), the blood glucose of the mice was measured again, and recorded as G15, G30, G45, G60 and G120, respectively.

### (2) Detection of insulin tolerance (ITT)

1. Fasting blood glucose of mice was detected by Byino blood glucose meter and blood glucose test strip, which was recorded as G0.
2. Mice were intraperitoneally injected with short-acting insulin at a dose of 0. 1U/g mice. Immediately after injection, the time at the moment was recorded as T0.
3. After 15 min(T15), 30 min(T30), 45 min(T45), 60 min(T60) and 120 min(T120), the blood glucose of the mice was measured again and recorded as G15, G30, G45, G60 and G120, respectively.

### (3) Detection of metabolites in mouse serum

1. Mouse blood was taken and left for 30min before centrifugation. The centrifugation condition was set at 300g for 30min. The upper serum was aspirated for later use.
2. ELISA kit was used to detect the serum insulin content and total cholesterol content in peripheral blood of mice.

According to the above values, analysis and comparison are carried out, and the results are shown in Figure 7.

Figure 7 shows that human derived muscle stem cells via tail vein injection significantly reduced fasting blood glucose in mice fed a high-fat diet. After intraperitoneal injection of glucose solution, the blood glucose level of mice increased. Along with the body self-metabolism, the rate of hypoglycemia in the treatment group was significantly higher than that in the model group, indicating that the treatment group had a better ability of hypoglycemia. After injection of short-acting insulin, the blood glucose of mice in control group and treatment group decreased rapidly and rose slowly, and the two groups kept basically the same. However, blood glucose of mice in the model group decreased slowly and then rose rapidly, indicating that mice in the treatment group were more sensitive to insulin than those in the model group and could quickly respond to the hypoglycemic effect of insulin, and the sensitivity of mice in the treatment group to insulin was similar to that of mice in the control group. Moreover, in the peripheral blood, human-derived muscle stem cells can increase insulin levels.

In conclusion, human-derived muscle stem cells have a good improvement effect on glucose metabolism in mice.

In peripheral blood, the content of total cholesterol in the treatment group was significantly lower than that in the model group, indicating that human-derived muscle stem cells also have a certain regulatory function on lipid metabolism.

### Example 8 Detection of adipose tissue Homeostasis in mice

### (1) Comparison of adipose tissue weights

The body weight, subcutaneous white adipose tissue weight and abdominal white adipose tissue weight of the model mice constructed in Example 6 were weighed respectively. And the analysis was calculated according to the weighing value.

### (2) Comparison of adipocyte hypertrophy degree

1. The mice were sacrificed under anesthesia, and the abdominal adipose tissue was obtained, and the tissue was put into 4% PFA (paraformaldehyde) for fixation for no less than 24 hours.
2. The adipose tissue was dehydrated, embedded in paraffin, and sectioned in paraffin with a thickness of 3µm.
3. The paraffin sections were stained with HE, sealed with gum, observed under a microscope and photographed.
4. Image Pro software was used to analyze images.

### (3) Expression changes of brown and white fatty genes

1. The abdominal tissue of mice was obtained, and the bean-sized of abdominal tissue was placed in the EP tube, and Trizol was added.
2. Tissue homogenizer was used to grind tissue mass. After grinding thoroughly, EP tube was put directly into -80°C refrigerator for frozen storage.
3. The frozen EP tube was taken out, and the upper layer of white fat mass on was scrapped off. After trizol was thawed, RNA in the sample was extracted.
4. The collected RNA was reverse transcribed by reverse transcription kit, and the target gene was subjected to QPCR and comparative analysis.

The result is shown in Figure 8.

Figure 8 shows that the subcutaneous fat weight and epididymal fat weight of mice in the treatment group decreased significantly compared with the model group. Moreover, human-derived muscle stem cells can significantly reduce the area of single adipocytes in adipose tissue. Compared with the model group, the expression of brown fat expression-related genes *ucp1*, *tbxc1* and *pgc1α* was increased, and the expression of white fat expression-related genes *leptin, fabp4* and *ppary* was decreased in the muscle stem cell treatment group. So muscle stem cells maintain the homeostasis of adipose tissue.

### Example 9: Detection of fatty lesions in mouse liver tissue

### (1) Comparison of liver tissue weight and appearance

The liver tissue of the model mice constructed in Example 6 was weighed, and the liver tissue of each group was photographed.

### (2) The degree of fatty infiltration in liver tissue was detected

1. Mouse liver tissue was obtained, fixed for dehydration, then embedded in paraffin, and sectioned in paraffin.
2. The paraffin sections were stained with HE, sealed with gum, and observed under a microscope and photographed.

The experimental results are shown in Figure 9.

Figure 9 shows that the liver tissue weight of mice in the treatment group was significantly reduced after intervention of human-derived muscle stem cells. Photographs of liver tissue showed that the liver tissue in the model group showed obvious fatty, but the degree of fatty of liver tissue in mice of the treatment group was improved. Similarly, liver section staining showed that human-derived muscle stem cells could reduce the fatty infiltration of liver tissue and reduce the degree of fatty liver disease.

### Example 10 Detection of inflammation level in vivo in mice

Peripheral blood of the model mice constructed in Example 6 was obtained and left for 30min before centrifugation. The centrifugation condition was set at 300g for 30min. After centrifugation, the serum was aspirated. Levels of TNF-α and IL-10 in serum were detected according to ELISA instructions.

The result is shown in Figure 10.

Figure 10 shows that the expression of inflammatory factors TNF-α and IL-1β in peripheral blood of mice in the model group is at a high level. TNF-α levels decreased significantly after exogenous administration of muscle stem cells. For the anti-inflammatory cytokine IL-10 with immune-modulatory function, the expression level of IL-10 increased significantly after human-derived muscle stem cell infusion. These results indicate that human-derived muscle stem cells can reduce the expression of TNF-α and increase the level of IL-10 *in vivo,* thereby reducing the level of inflammation in the body.

### Example 11: Effect of autologous muscle stem cells on blood glucose regulation in hyperglycemic monkeys

### (1) Extraction, proliferation and identification of monkey-derived muscle stem cells

1. Spontaneous hyperglycemic monkeys were obtained and anesthetized. The muscle tissue of the hind limb was taken, the size of the tissue was 1cm^{∗}1cm^{∗}1cm, and flow cytometry was used to extract and obtain monkey-derived muscle stem cells.
2. The extracted muscle stem cells were subjected to proliferation and cultured and expanded *in vitro.*
3. The proliferating cells were stained with stem cell surface characteristic antibodies CD31, CD34, CD45, CD29, EGFR and intracellular stem cell characteristic protein PAX7, and analyzed by flow cytometry analyzer.

### (2) Autologous infusion of monkey-derived muscle stem cells

1. Intravenous infusion of stem cell preparation, in which the cell infusion dose was 1-5×10⁶ cells /Kg of experimental monkeys.
2. After cell infusion, the fasting blood glucose of each monkey was measured every three days, and the fluctuation of blood glucose of each monkey was recorded.
3. The frequency of muscle stem cell infusion was once every two weeks for a total of 3-6 times.

### (3) Fasting blood glucose monitoring of experimental monkeys

Fasting blood glucose in the experimental monkeys was measured before and after muscle stem cell infusion, and the results were compared longitudinally.

The result is shown in Figure 11.

Figure 11 shows that the isolated monkey derived muscle stem cells highly expressed the muscle stem cell marker PAX7. The isolated cells were endothelial cell marker CD31 negative cells, hematopoietic stem cell marker CD34 negative cells, and leukocyte marker CD45 negative cells. The isolated cells highly expressed the stem cell marker CD29, and the expression ratio was 100%. The cells highly expressed the epidermal growth factor receptor EGFR by 90%, and CD56 up to 100% respectively. Therefore, the isolated cells were CD31⁻, CD34⁻, CD45⁻, CD29⁺, EGFR⁺, CD56⁺ and PAX 7⁺ muscle stem cells. The longitudinal comparison of blood glucose value of each experimental monkey showed that the fasting blood glucose of the experimental monkey was decreased after each infusion of autologous muscle stem cells after a long period of hyperglycemia. After injecting autologous muscle stem cells to spontaneously diabetic monkeys for 3-6 times continuously, the fasting blood glucose of the experimental monkeys remained at the normal level. Even when the muscle stem cell infusion was stopped, the experimental monkeys maintained normal blood sugar levels for a long time. Therefore, monkey autologous muscle stem cells can significantly improve the level of glucose metabolism, treat spontaneous hyperglycemia disease and maintain long-term efficacy.

### Example 12 Therapeutic effect of allogeneic murine stem cells on diabetic mice induced by high-fat diet

### (1) Establishment of diabetic mouse model induced by high-fat diet and in vivo transplantation of muscle stem cells derived from Balb/C mice

1. Male C57/BL mice aged 4 weeks were randomly selected and divided into groups. The mice were fed a normal diet containing 10% Kal fat and a high-fat diet containing 60% Kal fat, respectively, the weight of the mice was recorded and a weight change curve was formed. After 2-3 months of modeling, mice with a body weight of about 40 g were selected for grouping and experiment, and the body weight of each mouse was continuously recorded. High-fat fed mice developed insulin tolerance due to obesity, thus mimicking the human type 2 diabetes model.
2. After the establishment of the model, the mice were divided into three groups: control group (normal diet group), model group (high-fat diet group) and treatment group (high-fat diet group injected with Balb/C mouse muscle stem cells). Then the experiment was ready to begin.
3. The control group and the model group were injected with 200µL of the injection system sterile PBS every week by tail vein injection. The treatment group was injected with Balb/C murine-derived muscle stem cells, the injection volume was 1-5×10⁵ cells per mouse, and the injection system was 200µL. The injection cycle was once a week, and a total of 6 injections were given.

### (2) Detection of glucose tolerance (GTT)

4. Mice were given a treatment of fasting without water abstinence 15 hours before the test.
5. Fasting blood glucose of mice was detected by Bynoe blood glucose meter and blood glucose test strip, which was recorded as G0.
6. Mice were intraperitoneally injected with glucose at a dose of 1g/g mice. Immediately after injection, the time at the moment was recorded as T0.

After 15 min(T15), 30 min(T30), 45 min(T45), 60 min(T60) and 120min(T120), the blood glucose of the mice was measured again and recorded as G15, G30, G45, G60 and G120, respectively.

### (3) Detection of insulin tolerance (ITT)

1. Fasting blood glucose of mice was detected by Byino blood glucose meter and blood glucose test strip, which was recorded as G0.
2. Mice were intraperitoneally injected with short-acting insulin at a dose of 0.1 U/g mice. Immediately after injection, the time at the moment was recorded as T0.
3. After 15 min(T15), 30 min(T30), 45 min(T45), 60 min(T60) and 120min(T120), the blood glucose of the mice was measured again and recorded as G15, G30, G45, G60 and G120, respectively.

The hypoglycemic ability of mice was detected by glucose tolerance and insulin tolerance test, so as to evaluate the glucose metabolism level of mice.

### (4) Adipose tissue weight detection

The subcutaneous adipose tissue of mice in each group was separated, weighed and detected, and the weight of adipose tissue in each group was compared.

### (5) Weight weighing of liver tissue

In the body with type 2 diabetes caused by obesity, the liver tissue is complicated with fatty infiltration, forming fatty liver. The liver tissue of mice was removed and weighed for detection, and the weight of liver tissue of each group was compared.

### (6) Detection of mouse metabolites

1. Peripheral blood of mice was collected and left for 30min before centrifugation. The centrifugation condition was set at 300g for 30min.
2. Inflammatory cytokines IL-6 and IL-1β kit were used to detect the level of inflammation in mice.

The result is shown in Figure 12.

Balb/C murine-derived muscle stem cells via tail vein injection significantly reduced body weight and fasting blood glucose in mice fed a high-fat diet. After intraperitoneal injection of glucose solution, the blood glucose level of mice increased. Along with the body self-metabolism, the rate of hypoglycemia in the mice in the treatment group was significantly higher than that in the model group, indicating that the mice in treatment group had a better ability of hypoglycemia. The intervention of muscle stem cells derived from Balb/C mice reduced the fasting blood glucose of high-fat diet induced hyperglycemia mice, and enhanced the glucose metabolism ability of the model mice. Balb/C murine-derived muscle stem cells reduced the weight of subcutaneous fat, epididymal and inguinal fat, and liver tissue in obese mice. Moreover, in the peripheral blood, the level of peripheral inflammation in the model mice decreased significantly after infusion of Balb/C murine derived muscle stem cells.

All literatures mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. A use of a muscle stem cell for the preparation of a pharmaceutical composition for prevention, alleviation and/or treatment of obesity or metabolic disorders.

2. The use of claim 1, wherein the pharmaceutical composition is also used in one or more applications selected from the following group:
(a) Reduction in body weight;
(b) Improvement of glucose tolerance and/or insulin tolerance;
(c) Reduction in blood sugar;
(d) Improvement of insulin sensitivity;
(e) Reduction in fat content;
(f) Reduction in adipocyte weight;
(g) Reduction in adipocyte volume;
(h) Reduction in total cholesterol;
(i) Differentially regulating the expression levels of genes related to fat expression;
(j) Improvement of the expression level of genes related to brown fat expression;
(k) Reduction in the expression levels of genes related to white adipose expression;
(l) Maintenance of adipose tissue homeostasis;
(m) Reduction in liver tissue weight;
(n) Reduction in fatty infiltration of liver tissue;
(o) Reduction in the extent of fatty liver disease;
(p) Reduction in the level of inflammation of the body;
(q) Reduction in the expression of TNF-α and IL-1β in the body;
(r) Improvement of the expression of IL-10 in the body;
(s) Improvement of body metabolism;
(x) Improvement of muscle mass and strength.

3. The use of claim 1, wherein the metabolic disorders are selected from the following groups: diabetes mellitus, fatty liver disease, hypercholesterolemia, insulin resistance disease, hyperglycemia disease.

4. The use of claim 1, wherein the muscle stem cell is selected from the following groups: murine-derived muscle stem cell, human-derived muscle stem cell, monkey-derived muscle stem cell, dog-derived muscle stem cell, cat-derived muscle stem cell, horse-derived muscle stem cell, and a combination thereof.

5. The use of claim 4, wherein the human-derived muscle stem cell is a CD31⁻, CD34⁻, CD45⁻, CD29⁺, EGFR⁺ and PAX7⁺ cell.

6. The use of claim 1, wherein the diabetes mellitus is selected from the following groups: type 1 diabetes mellitus, type 2 diabetes mellitus.

7. The use of claim 2, wherein the improvement of muscle volume and strength is selected from the following groups: improvement of muscle support and enhancing athletic ability.

8. The use of claim 1, wherein the muscle stem cell is autologous, allogeneic, or xenogeneic.

9. The use of claim 1, wherein the muscle stem cell is extracted from the limbs or trunk muscles of the body and obtained by cell sorting.

10. A pharmaceutical composition for human or animal comprising:
(i) a muscle stem cell as a first active ingredient;
(ii) a second active ingredient that regulates glucose metabolism ;and
(iii) a pharmaceutically acceptable carrier.

11. The composition of claim 10, wherein the composition is used for one or more applications selected from the following groups:
(a) Reduction in body weight;
(b) Improvement of glucose tolerance and/or insulin tolerance;
(c) Reduction in blood sugar;
(d) Improvement of insulin sensitivity;
(e) Reduction in fat content;
(f) Reduction in adipocyte weight;
(g) Reduction in adipocyte volume;
(h) Reduction in total cholesterol;
(i) Differentially regulating the expression levels of genes related to fat expression;
(j) Improvement of the expression level of genes related to brown fat expression;
(k) Reduction in the expression levels of genes related to white adipose expression;
(l) Maintenance of adipose tissue homeostasis;
(m) Reduction in liver tissue weight;
(n) Reduction in fatty infiltration of liver tissue;
(o) Reduction in the extent of fatty liver disease;
(p) Reduction in the level of inflammation of the body;
(q) Reduction in the expression of TNF-α and IL-1β in the body;
(r) Improvement of the expression of IL-10 in the body;
(s) Improvement of body metabolism;
(t) Prevention, alleviation and/or treatment of obesity;
(u) Prevention, alleviation and/or treatment of diabetes;
(v) Prevention, alleviation and/or treatment of insulin resistance;
(w) Prevention, alleviation and/or treatment of metabolic disorders;
(x) Improvement of muscle mass and strength.

12. The composition of claim 10, wherein the second active ingredient is selected from the following group:
biguanides, sulfonylureas, non-sulfonylureas insulin secretagogues, thiazolidinedione insulin sensitizers, glycosidase inhibitors, insulin, glucagon-like peptide-1 analogues or agonists.

13. The composition of claim 10, wherein the composition is a drug that prevents and/or treats type 1 diabetes mellitus and type 2 diabetes mellitus.

14. The composition of claim 10, wherein the dosage form of the pharmaceutical composition is injection or lyophilized agent.
